# EUROPEAN PATENT APPLICATION

(11) **EP 1 645 597 A1**
(43) Date of publication of application: **12.04.2006**
(21) Application number: 04747628.8
(22) Date of filing: 09.07.2004
(51) Int. Cl.: C08L 101/14, C08K 7/24, A01N 25/10, A01N 25/12, A01N 43/12, A61K 47/34, A61K 47/04, A61K 9/10

(54) **CONJUGATE OF FINE POROUS PARTICLES WITH POLYMER MOLECULES AND UTILIZATION THEREOF**

(30) Priority: 09.07.2003 JP 2003293242
(71) Applicant: Tokyo University of Science, Educational Foundation, Tokyo 162-8601 (JP)
(72) Inventor: NAGASAKI, Yukio, Moriya-shi, Ibaraki 302-0128 (JP); TAKAHASHI, Tadahito, Yokohama-shi, Kanagawa 231-0852 (JP); KATAOKA, Kazunori, Nakano-ku, Tokyo 165-0031 (JP); YAMADA, Yoshiaki, Yokosuka-shi, Kanagawa 237-0062 (JP)
(74) Representative: Albrecht, Thomas
(86) International application number: PCT/JP2004/010162
(87) International publication number: WO 2005/005548

(57) **Abstract**

A conjugate which comprises fine porous particles (on which functional substance may be adhered) and a polymer based on water-soluble high molecular chain segment, and the use of the conjugate (for the purpose of stably supporting, or slowly releasing, a physiologically active substance, or the like).

## Description

### Technical Field

This invention relates to conjugate (or composite) of fine porous particles with polymer molecules which is usable in the field of medicines, cosmetics, diagnostic drugs, agriculture, prevention of environmental pollution, etc., and also to the utilization of said conjugate.

### Background Art

With use of their surface properties, fine porous particles such as silica and clay have been used as absorbent for separating various materials, or as a carrier for stabilizing functional substances. Also modification has been conducted with a view to improving particle properties. For instance, Document 1 (as shown below, together with the other documents) discloses calcined porous glass on the surface of which fluoroscein has been adsorbed. Document 2 discloses silica on the surface of which some kinds of dyes have been adsorbed, said silica having been precipitated from matasilicate solution with use of carbon dioxide in the presence of ethylene glycol, and having been modified with silane coupling agent having amino group.

Document 3 discloses magnetic bio/nano reactor made of latex particles of several hundreds nano meters on which high molecular electrolytes have been applied alternately so that finer multilayer of silica or magnetite and glucose oxidase (GOx) may be deposited thereon. Document 3 also teaches that such reactor shows enzymatic activity in proportion to the amount of GOx in the multilayer. Document 4 mentions fine silica particles on the surface of which steroids have been incorporated through the adsorption of cationic surface active agent. Document 4 also suggests that the bondability of steroids varies depending on the concentration of surface active agent. Document 5 discloses particles of silica-drug hybrid into which phenytoin has been incorporated during or after the hydrolysis and polycondensation of tetraethylene silicate. Controlled release of phenytoin from said hybrid is also considered in Document 5.

Document 6 discloses soft gelatin capsule which has encapsulated therein an adsorbate which comprises flake-like particles of magnesium metasilicate, SiO₂ or a mixture thereof, on which drug (such as antihistamine) has been adsorbed. It is also mentioned in Document 6 that said adsorbate serves to enhance the dispersion stability of drugs.

Document 7 discloses both inorganic matrix which has a slot and particles which contain drug such as pheromone. It is also mentioned that said particles are capable of slow release of drug.

### List of Documents

- Document 1:: Fujii, T. *et al.*, Research on Chemical Intermediates (1992), 17 (1), 1-14
- Document 2:: Krysztafkiewicz, A. *et al*., Applied Surface Science (2002), 199 (1-4), 31-39
- Document 3:: Fang, M. *et al*., Langmuir (2002), 18 (16), 6338-6344
- Document 4:: Cherkaoui, I. *et al*., International Journal of Pharmaceutics (1998), 176 (1), 111-120
- Document 5:: Goto, H. *et al*., Journal of Nanoparticle Research (1999), 1 (2), 205-213
- Document 6:: Lech, S. US 6027746A
- Document 7:: Anderson, T. et al., US 2003031694 A1

### Disclosure of Invention

Most of the aforementioned conventional particles have poor dispersion stability in aqueous fluid, in particular biological fluid such as blood, although there are exceptions like the adsorbate mentioned in Document 6 which is uniformly dispersible in liquid.

Thus, the inventors of this invention studied for the purpose of developing a system which enhances the dispersion stability, in aqueous fluid, of porous particles supporting functional substance thereon, and which, where necessary, is capable of the controlled release (slow release) of the supported functional substance from said porous particles into aqueous fluid. As a result, they have found out quite unexpectedly not only that porous particles surprisingly improve in dispersion stability in aqueous fluid when their surface is modified with specific polymer, but also that functional substance (if there is any) adsorbed is slowly released into aqueous fluid, and that, if there is no functional substance adsorbed thereon, the particles are able to remove, by adsorption, functional substance (injurious material) from a solution which contains the same.

Thus, this invention provides a conjugate which comprises fine porous particles on which functional substance is adhered or not adhered, and, immobilized on the surface of the particles, polymer molecule based on water-soluble high molecular chain segment.

In another embodiment, this invention provides a composition to slowly release functional substance, which comprises as an effective ingredient the above-mentioned conjugate wherein functional substance is adsorbed on the surface of fine porous particles, said functional substance selected from the group consisting of physiologically active substance, perfume, antimicrobial agent and raw materials for cosmetics. In this connection, this invention also provides the use of said conjugate for the purpose of the preparation of a composition to slowly release said functional substance, and also a method for expressing the action of said functional substance by administering said conjugate to a site or place which needs the administration of said functional substance.

In another embodiment, this invention provides a composition comprising the above-mentioned conjugate having no functional substance, by which to adsorb and remove functional substance, in particular harmful substance, which exists in aqueous solution or fluid. In this connection, this invention also provides a method to remove harmful substance which comprises setting said conjugate on a site or place which needs the removal of harmful substance.

In the following, this invention is explained in more detail.

For the fine porous particles which constitute the conjugate or composite of this invention, any particles are usable whether they may be originated in organic material, inorganic material or hybrid thereof so long as they have pores of such a diameter that functional substance as mentioned below is specifically adhered by some surface energy on the interface. The pores therefore have a diameter of 10 µm to 1 nm (10 Å), preferably 1 µm to 1 nm, more desirably 0.5 µm to 1 nm, although not restrictive. Average particle size, on the other hand, is required to be such that the objective of this invention is achieved, or that stable dispersion is accomplished by surface modification. Generally, average particle size is 5 nm to 10 mm, preferably 5 nm to 50 µm, more desirably 5 nm to 3 µm.

Examples of fine porous particles originated from organic material include crosslinked polystyrene, agarose, Sephadex, sintered continuous porous polyethylene and sintered continuous porous polypropylene.

Fine porous particles originated from inorganic material are preferably used in this invention. Examples thereof include, although not restrictive, silica, clay, zeolite, titania, alumina, zirconia, etc. Incidentally, silica in this invention does not simply refer to silicon dioxide. In this invention, the term of silica includes not only silicon dioxide but also any other porous fine particles as mentioned above that contain some content of silicon. Hence, silica particles which are formed by the processes as disclosed in the above-mentioned Documents 2 to 6 are also included in the silica of this invention. Clay includes not only inorganic natural products but also products processed therefrom. Mainly, however, clay includes clay mineral, and may include also coarse particle mineral such as quartz and feldspar, aluminum hydroxide, iron hydroxide and amorphous materials. Particles which are prepared from amorphous, crystalline and laminar-structured clay mineral are also included in the clay of this invention. Zeolite also includes both natural and synthesized products. Preferable examples of zeolite in this invention are the ones which are used as molecular sieve or adsorbent. Also titania, alumina and zirconia have wide range of concept in this invention.

Polymer molecule based on, or having as a main portion, water-soluble high molecular chain segment which constitutes the conjugate or composite of this invention is essentially required to have not only said water-soluble high molecular chain segment but also functional group or functional part by which said polymer molecule can be immobilized on the above-mentioned fine porous particles.

Any species of water-soluble high molecular chain segment is included in this invention so long as it works to achieve the objective of this invention, or to enhance the dispersion stability of the above-mentioned porous fine particles in aqueous fluid. Generally, however, polyethylene glycol (hereinafter sometimes abbreviated as PEG), polyvinyl pyrrolidone, polyvinyl alcohol, polyacrylamide and polymethacrylamide can be mentioned as preferable examples of water-soluble high molecular chain segment. The above-mentioned functional group or functional part which is contained in said polymer molecule works to immobilize said polymer molecule onto the surface of fine porous particles. The term "immobilize" is herein intended to mean that polymer molecule which is bonded onto the surface of fine porous particles cannot be removed. Said "bond" includes covalent bond between functional group (e.g., silanol, hydroxyl, mercapto, amino, carboxyl group, etc.) which may exist on, or may be added where necessary to, the surface of said particles and functional group or functional part in said polymer molecule. Said "bond" also includes physical interaction (e.g., electrostatic interaction, hydrophobic interaction, hydrogen bond, van der Waals force, etc.) between the surface of said particles and functional group, preferably functional part, in said polymer molecule.

For the sake of conciseness, a polymer which has PEG-originated segment which is one of preferable embodiments of water-soluble high molecular chain segment is mainly taken as an example of the aforementioned polymer molecule in the following explanation. Other polymer molecules will also be understood on the basis of the following explanation. Polymer (or polymer molecule) which has PEG-originated segment is represented by formula (I) as follows:

R¹-L₁-(CH₂CH₂O)ₙ-L₂-X (I)

wherein R¹ denotes hydrogen atom, methyl, formyl which may be protected, amino which may be protected, carboxy which may be protected, hydroxyl which may be protected or vinylsulfonyl group;
L₁ and L₂ independently denote valence bond or linker;
X denotes functional group or functional part to form covalent bond or a bond via physical interaction by which to immobilize said polymer molecule onto the surface of fine porous particles; and
n denotes an integer of 2 to 20,000.

Typical examples of L₁ as a linker include, although not restrictive, connecting groups as follows:

-(CH₂)ₚ-O-, -(CH₂)_{q}-COO- or -(CH₂)ᵣ-O-

wherein p, q and r each independently denote an integer of 0 to 8. These linkers are structurally incorporated, in the direction as mentioned, into the portion of L₁ in the above-mentioned formula (I).

Typical examples of L₂ as a linker include, although not restrictive, connecting groups as follows: 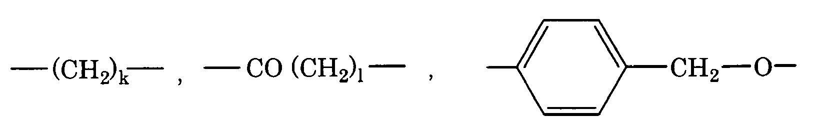 wherein k and 1 each denote an integer of 1 to 6. These linkers are structurally incorporated, in the direction as mentioned, into the portion of L₂ in the above-mentioned formula (I).

Examples of X as a functional group include silanol, mercapto, carboxyl and amino groups. When silanol group or hydroxyl group exists on the surface of fine porous particles, said group is condensed with silanol of X to form siloxane bond (-SiO-), or is condensed with carboxyl group of X to form ester bond (-OCO-), with which polymer molecule can be immobilized onto the surface of fine porous particles. X, on the other hand, may denote main chain portion of oligomer or polymer which has, on side chain, mono- or di-lower alkyl-substituted amino group; main chain portion of oligomer or polymer which has, on side chain, mercapto group; main chain portion of oligomer or polymer which has, on side chain, silanol group; main chain portion of oligomer or polymer which has, on side chain, carboxyl group; main chain portion of oligomer or polymer which has, on side chain, sulfo group; main chain portion of oligomer or polymer which has, on side chain, phosphate group; or main chain portion of oligomer or polymer which has, on side chain, hydroxyl group. In this specification, lower alkyl means straight chain or branched chain alkyl having one to six carbon atoms. Concrete examples of said lower alkyl include methyl, ethyl, *n*-propyl, isopropyl, butyl, *sec*-butyl and hexyl.

Main chain portion of oligomer or polymer which has, on side chain, mono- or di-lower alkyl-substituted amino group as a preferable embodiment of X is represented by formula (II) as follows: 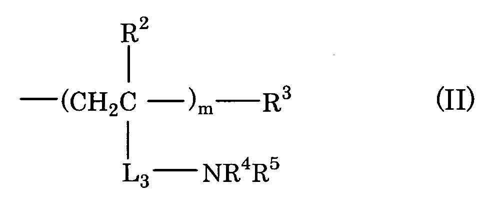 wherein R², R³, R⁴ and R⁵ each independently denote hydrogen atom or lower alkyl group, R³, in particular, denoting -(CH₂)ₓ-S-C (= S) N (alk)₂ wherein X denotes an integer of 0 to 6, alk denotes the same or different lower alkyl group;
L₃ denotes a group of formula as follows: 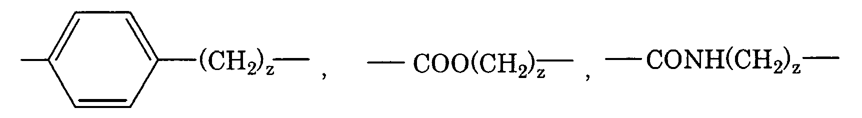 or -CONR⁶(CH₂)_{z}-
wherein Z denotes an integer of 0 to 10, R⁶ denotes a lower alkyl group which may be interrupted with oxygen atom, sulfur atom or nitrogen atom, and m denotes an integer of 1 to 2,000.

These polymers are produced by the method of, or a method based on the method of, Y. Nagasaki *et al*., Macromol. Chem. Rapid Commun. 1997, 18, 972. Polymers wherein R³ denotes -(CH₂)ₓ S-C(=S) N (alk)₂ and L₃ denotes 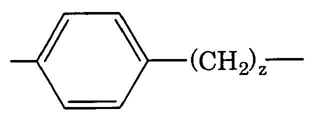 are produced in accordance with Japanese Patent Application No. 2003-049000. In certain methods for production as illustrated by the following reaction schemes, inifertor as mentioned below is used, and, furthermore, dialkylaminostyrene etc. are polymerized.

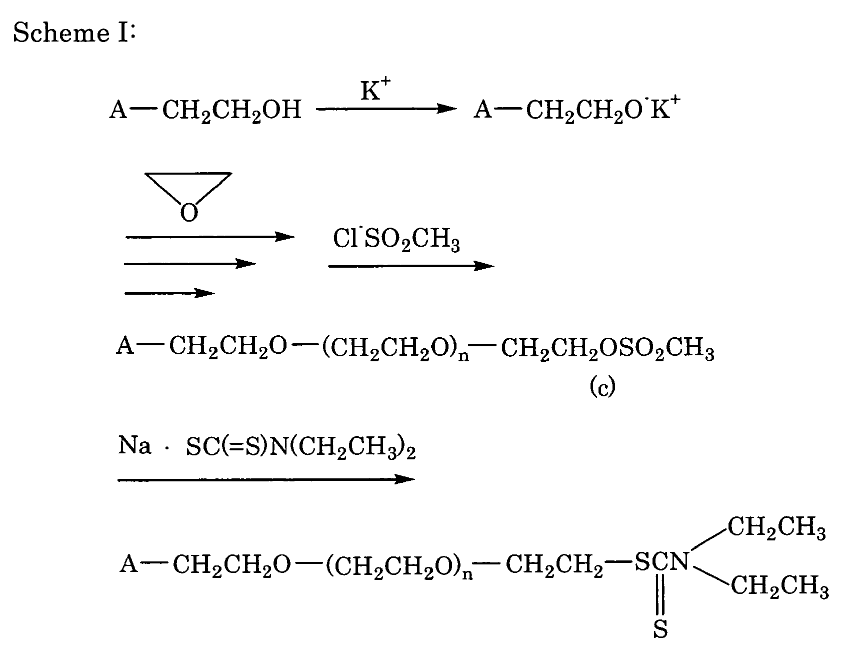

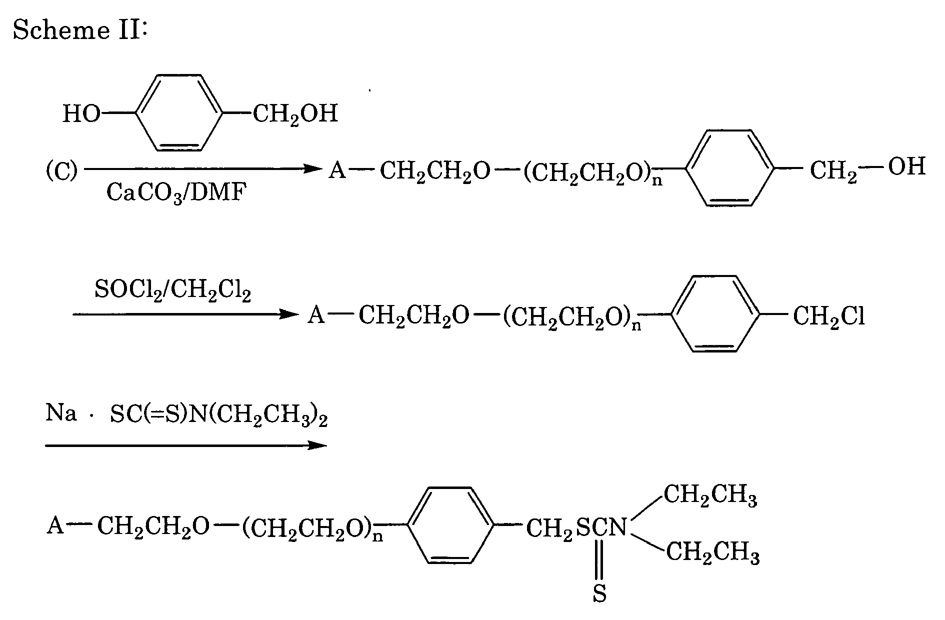

### (In the above formulae, A may denote lower alkyloxy, formyl which may be protected, hydroxy which may be protected, etc.)

Formyl which may be protected in the above-mentioned definitions of R¹ and A has a formula as follows: 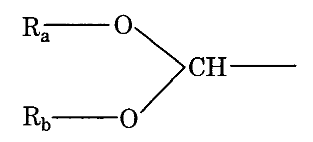 wherein Rₐ and R_{b}, taken separately, denote lower alkyl, and, taken together, denote methyl-substituted ethylene; or Rₐ-O and R_{b}-O, taken together, denote O = (in which case, formyl OCH- is shown). Amino which may be protected, carboxyl which may be protected and hydroxy which may be protected mean groups which are either protected by protective groups known in the field of peptide synthesis or the like, or unprotected. Moreover, protected amino group includes maleimide, and protective group for hydroxyl includes *p*-toluenesulfonyl group.

Polymers which have a functional group, i.e., "functional group or functional part to form covalent bond or a bond via physical interaction" as mentioned above, are obtained by selecting, as "A" in the above Scheme I, protected mercapto group (e.g., trialkylsilylsulfide), protected silanol group (e.g., trialkoxysilyl), etc., and conducting the first step of Scheme I, and, if necessary, detaching each protective group (see, for instance, Japanese Patent KOKAI Publication No. Hei 11-322917 and Japanese Patent KOKAI Publication No. 2003-149245). Similarly, polymers which have one of the other functional groups can be produced by following the above Scheme I, and by selecting, as "A", a corresponding protected functional group.

Polymers which have a main chain portion of oligomer or polymer which has, on side chain, a mono- or di-lower alkyl-substituted amino group are obtained by conducting a polymerization reaction to form a second block with use of (meth)acrylic acid ester or amide having a desired functional group (which may be protected) in place of dialkylaminoethyl(meth)acryloyl which is employed in the above-mentioned Y. Nagasaki *et al*., or by using the above-mentioned inifertor and further forming a second block with use of (meth)acrylic acid ester or amide having a desired functional group (which may be protected).

Polymers which have, in their molecule, a portion as represented by the above-mentioned formula (II) can, when the fine porous particles are silica, clay, zeolite or the like, be physically immobilized on the surface of said particles in an aqueous fluid, and, furthermore, can be kept immobilized and unseparated from the surface regardless of the change of ionic intensity etc. Besides, polymer molecule can be rendered temperature-sensitive if methyl or ethyl is selected for R⁴ and R⁵ in the above-mentioned formula (II) (e.g., polymer molecule can be rendered water-insoluble near a temperature of 40°C when L₃ denotes -COO(CH₂)₂ and R⁴ and R⁵ are methyl or ethyl). Moreover, the dispersibility of polymer molecule in an aqueous fluid can be controlled by choosing R⁴ and R⁵ and controlling hydrophilic-hydrophobic balance of polymer or p*K*a of amino group. Such a technique can be utilized for controlling the behavior of a functional substance, which has been adsorbed on fine porous particles in a conjugate of this invention, when it is released from conjugate into aqueous fluid. Aqueous fluid in this specification includes not only water *per se* but also biological fluid (e.g., blood and urine) and aqueous solution which contains buffer, isotonic agent or water-miscible organic solvent (e.g., methanol, ethanol, acetone, acetonitrile, dimethylformamide, etc.).

Examples of functional substance which can be adsorbed on fine porous particles are, although not restrictive, as follows:
(a) Dyes such as azo dye, quinine dye, triarylmethane dye, cyanine dye, phthalocyanine dye and indigo dye, each of which includes dyes which are well known to those skilled in the art.
(b) Fluorescent or luminescent substance such as pyrene, anthracene, FITC, Texas Red, CY-3, CY-5, CdS quantum dot and CdSe/ZnS quantum dot.
(c) Physiologically active substance such as Activin-A (human type), ALCAM (human type), ANG (angiogenin) (human type), BDNF (human type), BMF-2 (human type), Cadherin, Caspase-1 (human type), CD14, CD40, cytochrome C, EGF (human type), Fas ligand, each class of interleukin and each class of interferon.
(d) Perfume such as cis-3-hexenol and its esters, 3,6-nonadienol, jasmine and its analogues, lactones including delta-decalactone etc., 2-*n*-hexylcyclopentanone and prenylacetate. Also antibody, enzyme, nucleic acid and peptide nucleic acid are included in the above physiologically active substance.
(e) Magnetic body such as ferricolloid (Fe₂O₃).
(f) Food additive such as extract of *Araliae Cardatae Rhizoma*, extract of *Malvaceae*, extract of whole plant of azuki bean, erythorbic acid and γ-orynanol.
(g) Raw material for cosmetics such as ascorbic acid and its derivatives, acetyl pantothenyl ether, acerola extract, Gambir extract, abietic acid ester, allantoin, arbutin, ginkgo extract, fennel extract, *Matricaria chamomilla* extract, *Glycyrrhizae Radix* extract, kojic acid, tocopherol and its derivatives, salicylic acid and *Coicis Semen* extract.
(h) Antimicrobial agent such as antiviral agent and antibacterial agent, antifungal agent, antitumor agent and antibiotic. Examples of said antimicrobial agent include, although not restrictive, macrolide antibiotics such as azithromycin, erythromycin, josamycine, spiramycin and tylosin; tetracycline antibiotics such as oxytetracycline, oleomycine, chlortetracycline, tetracycline, doxycycline and minocycline; aminoglycoside antibiotics such as arbekacin, kanamycin, gentamycin, streptomycin, spectinomycin, neomycin and tobramycin; glycopeptide antibiotics such as avoparcin, teicoplanin and vancomycin; β-lactam antibiotics such as ampicillin, oxacillin, sulbactam, cephalosporin, cephatriazone, tazobactam, naphcillin, penicillin and meticillin; chloramphenicol antibiotics such as chloromycetin; streptogramin antibiotics such as virginiamycin and prystinamycin; other antibiotics such as linkomycin and daptomycin; sulfa drug such as sulfonamide; quinolone antifungal agent such as quinolone and nalidixic acid; new quinolone antifungal agent such as enrofloxacin, ofloxacin, sarafloxacin, cipro, tropane and baytril.

Such functional substances as mentioned above may be adsorbed on fine porous particles either before the formation of conjugate of fine porous particles and polymer molecule, or during or after the formation of conjugate. Hence, conjugate which contains no functional substance is usable for adsorbing and removing functional substance, in particular harmful substance (e.g., cholesterol and neutral fat), which exists in aqueous fluid.

Conjugate of fine porous particles and polymer molecule is produced by contacting fine porous particles and polymer molecule, in the presence of functional substance where necessary, for a time enough for the formation of conjugate in a solvent, e.g., water, tetrahydrofuran, lower alkanol, dichloromethane, supercritical carbon dioxide and aromatic hydrocarbon, which can dissolve polymer and also functional substance if any, at a temperature at which the solvent can maintain liquid state. Polymer molecule is usually used in an amount 1/100 to 10,000 times, preferably 1/10 to 1,000 times, more desirably 1 to 200 times, as much as fine porous particles on weight basis.

The amount of functional substance which is to be adsorbed on fine porous particles is 0.00001 % by weight to 200 % by weight, preferably 0.1 % by weight to 30 % by weight, more desirably 0.1 % by weight to 10 % by weight, based on the weight of fine porous particles, although it varies depending on combination.

When put in an aqueous fluid, thus produced conjugate of fine porous particles having functional substance adsorbed thereon and polymer molecule can detach functional substance from fine porous particles in a controlled manner depending on the change of temperature, the change of pH or the addition of water-miscible organic solvent, and release the functional substance into the aqueous fluid, if the functional substance is neither fluorescent or luminescent substance nor certain kind of dye.

Hence, if the functional substance is antimicrobial agent, in particular antifungas agent, the conjugate of this invention is capable of long-term sterilization when applied to water or waste water of industries which use a large amount of water, such as pulp industry, aluminum extension industry, food processing industry or aqueous lubricant industry. If the functional substance is physiologically active substance, the conjugate serves to cure or prevent disease when administered or implanted in necessary site of living body to slowly release physiologically active substance in the place. If, on the other hand, the functional substance is perfume, conjugate slowly releases perfume when put in aqueous fluid, and thus serves as an interior aromatic or aromatic cosmetics. Furthermore, when raw materials of cosmetics are lacking in stability or have adverse effects on skin, the conjugate of this invention serve for the stabilization or safety use of the raw materials of cosmetics.

### Brief Description of Drawings

Figure 1 shows a concept of dispersion stabilization of fine porous particles by this invention.
Figure 2 is a graph which shows the dispersion stability of conjugate of silica particles and PEG/PAMA block copolymer with regard to the change of ionic intensity. The plot with mark ■ shows the result of silica particles alone, and the plot with mark • shows the transmission of aqueous solution of conjugate.
Figure 3 is a graph which shows special properties of pyrene molecule when it is adsorbed onto conjugate of clay particles and PEG/PAMA block copolymer.
Figure 4 is a graph which shows the behavior of CdSe/ZnS type semiconductor nanoparticles when they are adsorbed onto a conjugate of clay particles and PEG/PAMA block copolymer.
Figure 5 is a graph which shows the result of antioxidation of hydroquinone adsorbed on a conjugate of clay particles and PEG/PAMA block copolymer with use of L-DOPA. The plot with mark ■ shows the result of hydroquinone alone. The plot with mark • shows the result of hydroquinone adsorbed on clay particles. The plot with mark ▲ shows the result of three-hour incubation of hydroquinone alone. The plot with mark ▼ shows the result of three-hour incubation of hydroquinone adsorbed on clay particles.
Figure 6 is a graph which shows the dispersion stability of conjugate of clay particles and PEG/PAST block copolymer.
Figure 7 is a graph which shows how pyrene which has been adsorbed on clay particles is released.
Figure 8 is a graph which shows the behavior of bovine serum albumin (BSA) when it is adsorbed onto conjugate of clay particles and PEG/PAMA block copolymer.

### Best Mode for Carrying Out the Invention

In the following, this invention will be explained with working examples, which are to facilitate the understanding of this invention.

### Example 1: How to produce silica particles conjugate

Polymer which is used in this Example 1 (abbreviated as PEG/PAMA; having the following formula)

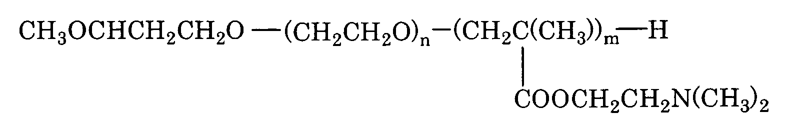

PEG (Mn = 4,000 g/mol) and PAMA (polymethacrylic acid (2-N,N-dimethylaminoethyl); Mn = 2,000) which had been obtained by the method as given in the above-mentioned Y. Nagasaki *et al*., Macromol. Chem. Rapid Commun. 1997, 18, 927 were used as a dispersion stabilizer, with which silica particles were modified.

To 150 µL of a colloidal silica solution (manufactured by Nissan Chemical Industries Co., Ltd.; Snowtex S® (colloidal silica); particle size: 17 nm) (containing 41.5 mg of silica particles) which had passed through a filter, 9.85 ml of phosphate buffer (pH = 7.4) was added, so that the total amount might be 10 ml. To the resultant mixture, ultrasonic wave was applied for 30 minutes, and, then, a solution of 200 mg of PEG/PAMA block polymer (4000/2000) dissolved in 10 ml of phosphate buffer was added. The change of transmission was examined by UV-Vis spectrum. As a result, the mixture was found to be almost completely transparent.

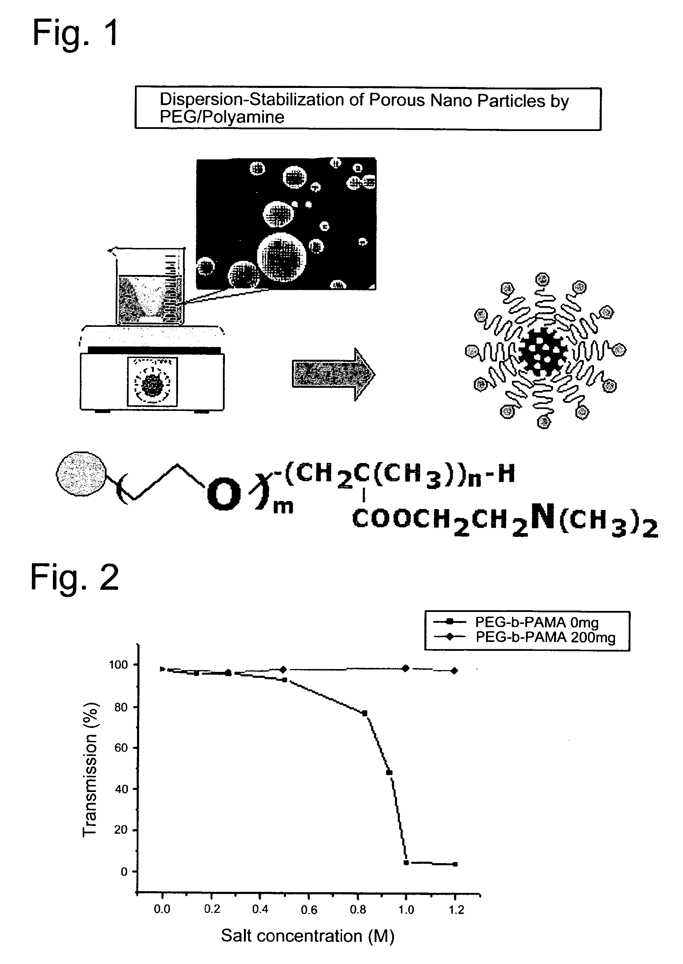

The stability of thus prepared conjugate was evaluated by changing ionic intensity. The results are shown in Figure 2. It is seen that, whereas a system which contains no block polymer easily agglomerates and precipitates and becomes cloudy when ionic intensity is enhanced, a system which contains block polymer blended keeps quite transparent even at a high concentration of salt.

From dynamic light scattering (DLS) measurement, particle size was found to be 70 nm.

### Example 2: How to produce a conjugate of clay particles with PEG/PAMA

Conjugate was prepared by the same method as in Example 1 except that silica was replaced with clay particles (Laporte Industries LTD; Laponite XLG-(F) 5 mg). The stability of thus prepared conjugate was evaluated by changing ionic intensity.
Clay-PEG/PAMA composite particles had excellent dispersion stability as in silica. From DLS measurement, particle size was found to be 140 nm.

### Example 3: How griseofulvin is supported on a silica-PEG/PAMA conjugate

In this Example 3, the conjugate as prepared in Example 1 was made to support griseofulvin as an antifungal agent. In acetone, 5 mg of griseofulvin (GF) was dissolved, and the resultant solution was added to a phosphate buffer solution which contained silica particles (40 mg), so that GF might be incorporated with silica particles. Subsequently, 200 mg of PEG/PAMA was added, and the resulting mixture was stirred to give a conjugate. As a result of fluorescence measurement, it was confirmed that 3 % of GF had been incorporated in the conjugate.

### Example 4: How pyrene is supported on a clay particles-PEG/PAMA conjugate

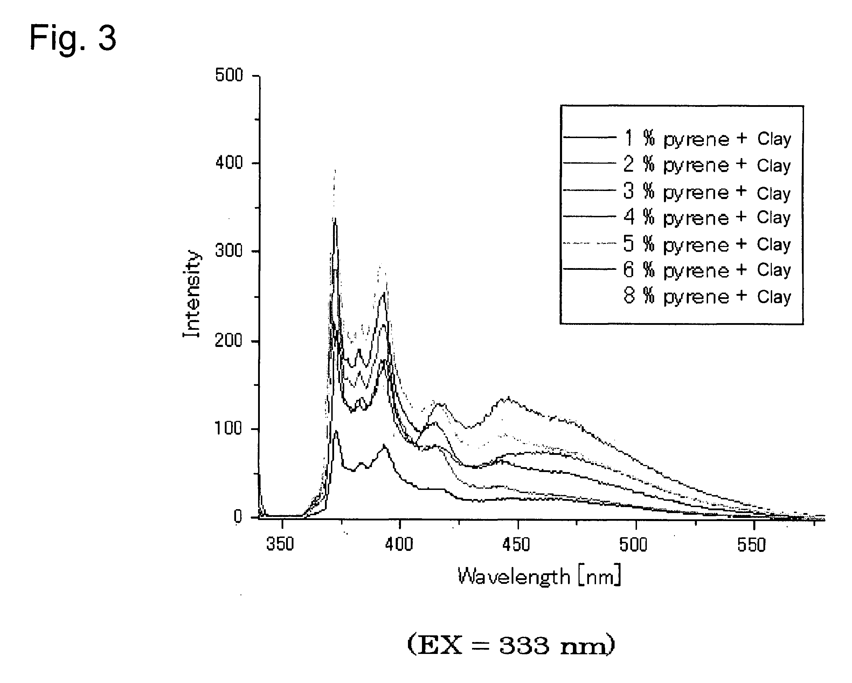

To 10 mL of a phosphate buffer (10 mM, pH 7.4; Na₂HPO₄.12H₂O, 2.865 g/l; NaH₂PO₄.2H₂O, 0.312 g/l), there were added 100 mg of clay particles and 200 mg of PEG/PAMA. To the resultant mixture, a solution of pyrene dissolved in acetone (1 mg to 8 mg/mL) was added, and the resultant mixture was stirred for one hour in darkness. Thus prepared solution was dialyzed so that unadsorbed pyrene might be removed, and was then subjected to fluorescence measurement. As is seen in Figure 3, fluorescent intensity gradually increased, and, when the concentration of pyrene exceeded 6 %, the absorption of excimer increased. It is known from this fact that pyrene had been condensed in pores. Thus, fluorescent body was stably supported on clay.

### Example 5: How semiconductor fluorescent particles are supported on a clay particles-PEG/PAMA conjugate

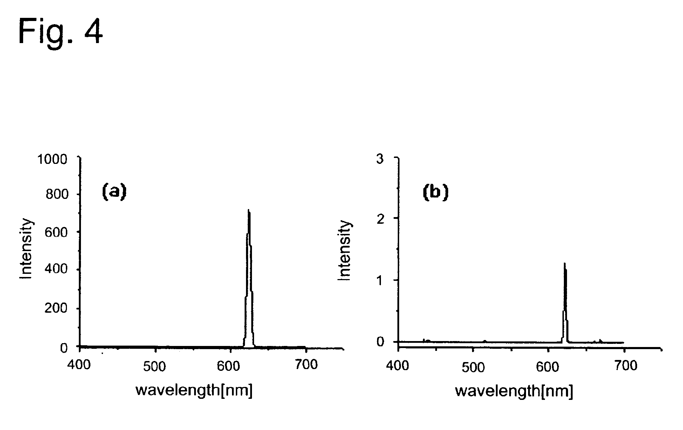

To 10 mL of chloroform, there were added 10 mg of clay, 0.13 ml (20 ·mol/ml) of CdSe quantum dot and 200 mg of PEG/PAMA (5000/2000). The resultant mixture was stirred well for two hours, and chloroform was distilled off. To the mixture, 10 mL of phosphate buffer was added, and, then, fluorescent measurement was conducted. Although CdSe particles *per se* which are supported on clay do not emit light in phosphate buffer, CdSe-clay which had been stabilized with PEG/PAMA emitted strong light (see Figure 4). Figure 4 shows the behavior of CdSe/ZnS type semiconductor nanoparticles when they are adsorbed onto the conjugate. Luminescent intensity remarkably lowers when CdSe/ZnS type semiconductor nanoparticles are adsorbed on clay (see (b)) whereas very strong light emission behavior is seen in the case of conjugate.

### Example 6: How hydroquinone is supported on a clay particles-PEG/PAMA conjugate

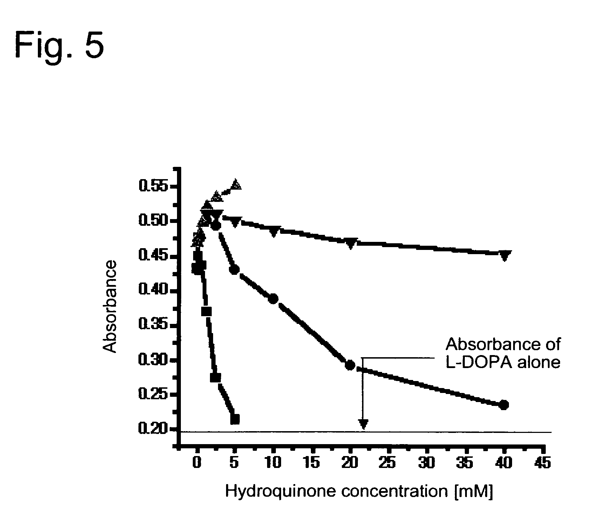

Hydroquinone was supported on conjugate in the same manner as in Example 4 except that hydroquinone was used in place of pyrene. Hydroquinone and clay particles-supported hydroquinone were examined for the resistance to the oxidation of L-DOPA. As is seen in Figure 5, preferable results were produced. In particular, whereas hydroquinone had completely lost antioxidation property when L-DOPA was added after three-hour incubation of hydroquinone in phosphate buffer, clay particles-supported hydroquinone still showed activity. It was thus confirmed that clay particles-supported substance had been successfully stabilized, and that the conjugate was excellent as a carrier for slow release of drugs.

### Example 7: Stabilization of fine porous particles by PEG/poly(4-dimethylaminomethylstyrene) (abbreviated as PEG/PAST)

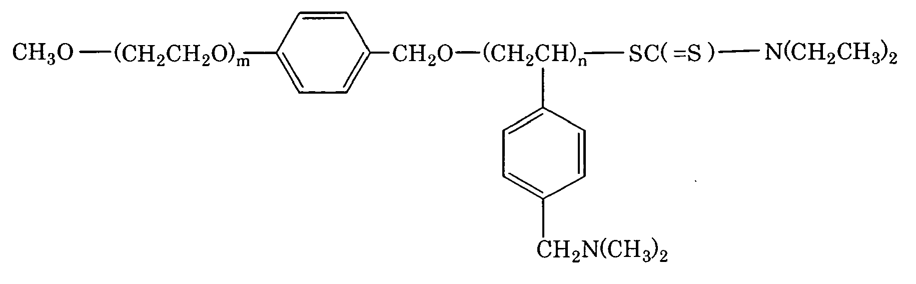

PEG/PAST was obtained by use of the final product of the above-mentioned Scheme II as an inifertor, and by subjecting said final product to anionic living polymerization with 4-dimethylaminoethylmethylstyrene. The process of Example 2 was repeated except that so produced PEG/PAST was used in place of PEG/PAMA, and, thus, a conjugate of clay particles with PEG/PAST was obtained.

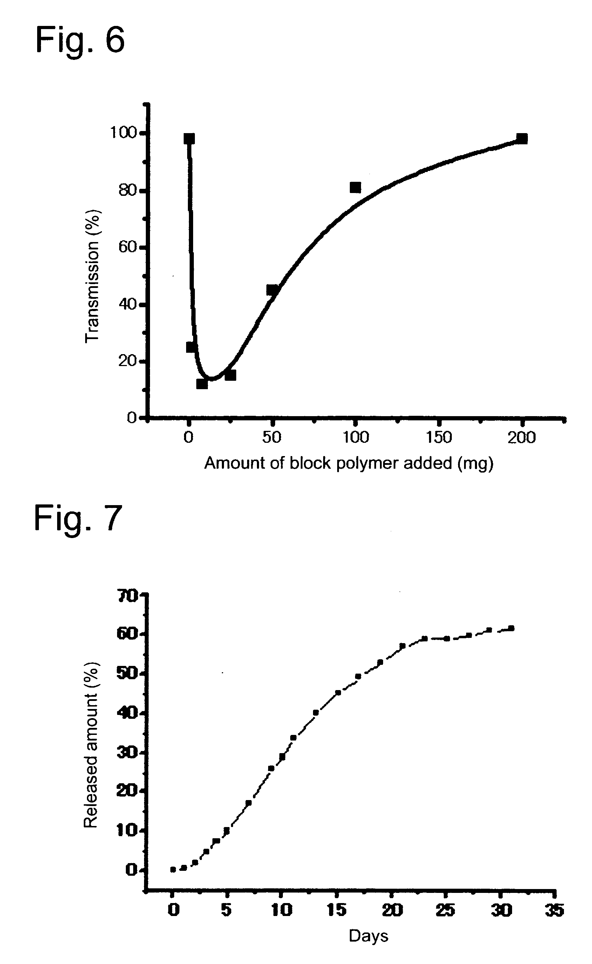

As is seen in Figure 6, when the amount of PEG/PAST added was increased, transparency was enhanced, and, thus, it was confirmed that conjugate was improved in dispersion stability.

### Example 8: Experiment on the release of pyrene

A 100 ml egg-shaped flask was fed with 6 mg of clay, 6 ml of acetone and 0.3 mg (19.8 ·l) (5 wt %) of a solution of pyrene dissolved in acetone. The resultant mixture was stirred for two hours so that pyrene might be incorporated with clay, and, then, acetone was removed from the mixture by evaporation. Subsequently, the mixture was passed through a 0.4 µm syringe filter, and, thus, pyrene which had not reacted with clay particles were removed. Thus obtained sample was wrapped in a dialysis membrane, which was then put in a 2-L sealable container. Then, 1 mL of the sample solution was taken out, and was subjected to fluorescent measurement. The change of fluorescent intensity was observed, and a comparison was made with calibration data which had previously been prepared, and, thus, the amount of pyrene released was calculated. Final results are shown in Figure 7. Apart from the above, pyrene-clay solution in the filter was subjected to fluorescent intensity measurement, and, thus, the state of pyrene coordinated with clay was examined. Incidentally, all the reactions were carried out in darkness.

From the amount of pyrene released from the dialysis membrane, it is known that almost no pyrene that had been incorporated with clay was released up to Day 2; from about Day 3, pyrene began to be released; then, up to about Day 20, pyrene was increasingly released; thereafter, pyrene was released in a very moderate manner; and at about Day 30, it is confirmed that almost no pyrene was released.

### Example 9: Evaluation of inhibition of non specific adsorption of protein

A 30-ml sample tube was fed with 660 µl of silica particles and 10 mL (0.15 M) of PBS, and, then, the resultant mixture was subjected to an ultrasonic wave treatment for 30 hours. Subsequently, 0 to 200 mg of PEG/PAMA was added for the purpose of sample adjustment, and, then, the sample was subjected to ultracentrifugation (70000 rpm, 312000 x G, 30 minutes) three times, and, thus, PEG/PAMA which had not reacted with clay was removed. Then, to the resultant sample, there was added a solution of FITC-BSA, i.e., fluorescence-labeled protein, dissolved in phosphate buffer so that the solution might have a concentration of 0.1 mg/ml. The resultant mixture was shaken for one hour, and was then subjected to ultracentrifugation (70000 rpm, 312000 x G, 30 minutes). Thus obtained supernatant liquid was subjected to fluorescent measurement (ex: 493 nm), and, thus, the amount of protein adsorbed was assayed.

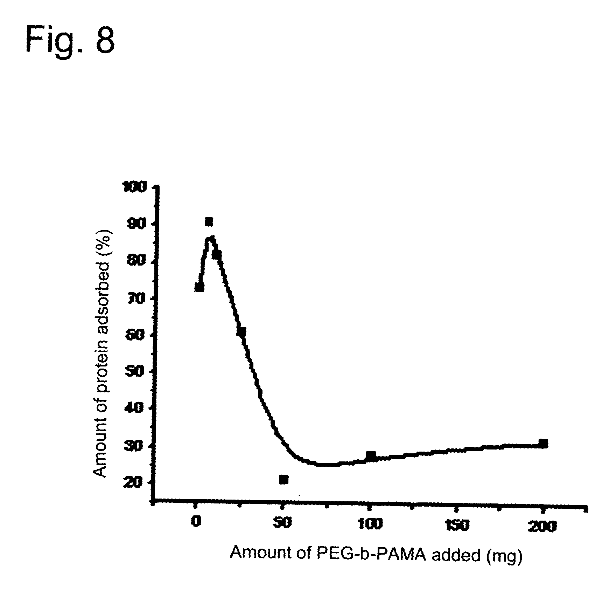

Figure 8 shows the data of the amount of protein adsorbed on silica, which data were obtained by assaying both the amount of protein in supernatant and the amount of protein peeled off silica particles. It is seen that, when there was little or no polymer, a large amount of protein was adsorbed on particles whereas, as the amount of polymer added was increased, non specific adsorption was effectively inhibited.

### Industrial Applicability

This invention provides a conjugate which supports various kinds of functional substances, and which can stably be dispersed in aqueous fluid. Industrial field to which the conjugate is applicable is broadened depending on the function of the functional substances. For instance, when the functional substance is a physiologically active substance, the conjugate is usable in industries for the manufacture of medicinal drugs.

## Claims

1. A conjugate which comprises fine porous particles on which functional substance is adhered or not adhered, and, immobilized on the surface of the particles, polymer molecule based on water-soluble high molecular chain segment.

2. A conjugate of claim 1 wherein the polymer molecule has, as a water-soluble high molecular chain, a chain originated in a polymer selected from the group consisting of polyethylene glycol, polyvinyl pyrrolidone, polyvinyl alcohol, polyacrylamide and polymethacrylamide, and wherein the polymer molecule also has functional group or functional part to form covalent bond or a bond via physical interaction by which to immobilize said polymer molecule itself onto the surface of fine porous particles.

3. A conjugate of claim 1 wherein the polymer molecule has formula (I) as follows:
R¹-L₁-(CH₂CH₂O)ₙ-L₂-X (I)
wherein R¹ denotes hydrogen atom, methyl, formyl which may be protected, amino which may be protected, carboxy which may be protected, hydroxyl which may be protected or vinylsulfonyl group;
L₁ and L₂ independently denote valence bond or linker;
X denotes functional group or functional part to form covalent bond or a bond via physical interaction by which to immobilize said polymer molecule onto the surface of fine porous particles; and
n denotes an integer of 2 to 20,000.

4. A conjugate of claim 3 wherein X is selected from the group consisting of mercapto group, silanol group, carboxyl group and amino group, or from the group consisting of main chain portion of oligomer or polymer which has, on side chain, mono- or di-lower alkyl-substituted amino group; main chain portion of oligomer or polymer which has, on side chain, mercapto group; main chain portion of oligomer or polymer which has, on side chain, silanol group; main chain portion of oligomer or polymer which has, on side chain, carboxyl group; main chain portion of oligomer or polymer which has, on side chain, sulfo group; main chain portion of oligomer or polymer which has, on side chain, phosphate group; and main chain portion of oligomer or polymer which has, on side chain, hydroxyl group.

5. A conjugate of claim 1 wherein the fine porous particles are organic or inorganic particles which have an average particle size of 10 nm to 10 mm, and have pores of a diameter of 10 µm to 1 nm (10 Å).

6. A conjugate of claim 5 wherein the fine porous particles are selected from the group consisting of crosslinked polystyrene, agarose, Sephadex, sintered continuous porous polyethylene and sintered continuous porous polypropylene.

7. A conjugate of claim 5 wherein the fine porous particles are selected from the group consisting of silica, clay, zeolite, titania, alumina and zirconia.

8. A conjugate of claim 1 wherein no functional substance is adsorbed on the fine porous particles.

9. A conjugate of claim 1 wherein the fine porous particles has functional substance adsorbed thereon.

10. A conjugate of claim 1 wherein the functional substance is adsorbed on the fine porous particles, and is selected from the group consisting of dye, fluorescent or luminescent substance, magnetic body, physiologically active substance, antimicrobial agent, food additive, perfume and raw material for cosmetics.

11. A composition for slowly releasing a physiologically active substance which composition comprises, as effective ingredient, a conjugate of claim 1 wherein a functional substance is adsorbed on fine porous particles, said functional substance being a physiologically active substance.

12. A composition for slowly releasing a perfume which composition comprises, as effective ingredient, a conjugate of claim 1 wherein a functional substance is adsorbed on fine porous particles, said functional substance being a perfume.

13. A composition for slowly releasing an antimicrobial agent which composition comprises, as effective ingredient, a conjugate of claim 1 wherein a functional substance is adsorbed on fine porous particles, said functional substance being an antimicrobial agent.

14. A composition for slowly releasing a raw material for cosmetics which composition comprises, as effective ingredient, a conjugate of claim 1 wherein a functional substance is adsorbed on fine porous particles, said functional substance being a raw material for cosmetics.

15. A composition for adsorbing and removing harmful substance which exists in aqueous solution, which composition comprises, as an effective ingredient, a conjugate of claim 1 which contains no functional substance.

16. A composition of claim 15 wherein the harmful substance is cholesterol or neutral fat, and wherein said conjugate is administered into living body.

17. A method for controlling microorganisms in an environment wherein microorganisms grow, which method comprises placing in said environment a conjugate of claim 1 wherein a functional substance is adsorbed on fine porous particles, said functional substance being an antimicrobial agent.

18. A method of makeup which comprises applying a cosmetic to a site of skin which needs raw material for the cosmetic, said cosmetic containing a conjugate of claim 1 wherein a functional substance is adsorbed on fine porous particles, and said functional substance being a raw material for cosmetics.
